# EUROPÄISCHE PATENTSCHRIFT

(11) **EP 2 188 236 B1**
(45) Veröffentlichungstag und Bekanntmachung des Hinweises auf die Patenterteilung: **23.10.2013**
(21) Anmeldenummer: 08863558.6
(22) Anmeldetag: 28.10.2008
(51) Int. Cl.: C07C 29/16, C07C 31/125, C07C 45/50, C07C 47/02

(54) **EINSTUFIGES KONTINUIERLICHES VERFAHREN ZUR HYDROFORMYLIERUNG VON HÖHEREN OLEFINEN ODER OLEFINGEMISCHEN**
ONE-STAGE CONTINUOUS PROCESS FOR HYDROFORMYLATING HIGHER OLEFINS OR OLEFIN MIXTURES
PROCEDE CONTINU EN UNE ETAPE POUR L'HYDROFORMYLATION D'OLEFINES OU DE MELANGES OLEFINIQUES SUPERIEURS

(30) Priorität: 20.12.2007 DE 102007061649
(43) Veröffentlichungstag der Anmeldung: 26.05.2010
(73) Patentinhaber: Evonik Degussa GmbH, 45128 Essen (DE)
(72) Erfinder: LÜKEN, Hans-Gerd, 45770 Marl (DE); DREES, Stefan, 48249 Dülmen (DE); KAIZIK, Alfred, 45772 Marl (DE); BÜSCHKEN, Wilfried, 45721 Haltern Am See (DE)
(86) Internationale Anmeldenummer: PCT/EP2008/064584
(87) Internationale Veröffentlichungsnummer: WO 2009/080396

(56) Entgegenhaltungen:
- EP-A- 0 850 905
- DE-A1- 19 939 491

## Beschreibung

Die vorliegende Erfindung betrifft ein einstufiges kontinuierliches Verfahren zur Hydroformylierung von höheren Olefinen oder Olefingemischen in Gegenwart von unmodifizierten Kobaltkatalysatoren, mit dem höhere Oxo-Aldehyde und/oder Alkohole erhalten werden können.

Höhere Alkohole, insbesondere solche mit 6 bis 25 Kohlenstoffatomen, können bekanntlich durch katalytische Hydroformylierung (Oxo-Reaktion) der um ein Kohlenstoffatom kürzeren Olefine und anschließende katalytische Hydrierung der Aldehyd- und Alkohol-haltigen Reaktionsgemische hergestellt werden. Sie werden vorwiegend für die Herstellung von Weichmachern und Detergentien verwendet. Es ist weiterhin aber auch möglich, durch Destillation der Hydroformylierungsgemische Aldehyde abzutrennen. Diese können beispielsweise zur Herstellung von Carbonsäuren genutzt werden.

Die Art des Katalysatorsystems und die optimalen Reaktionsbedingungen für die Hydroformylierung sind von der Reaktivität des eingesetzten Olefins abhängig. Die Abhängigkeit der Reaktivität der Olefine von ihrer Struktur ist z. B. von J. Falbe, New Syntheses with Carbon Monoxide, Springer-Verlag, Berlin, Heidelberg, New York, 1980, Seite 95 ff., beschrieben. Die unterschiedliche Reaktivität speziell der isomeren Octene ist ebenfalls bekannt (B. L. Haymore, A. van Hasselt, R. Beck, Annals of the New York Acad. Sci., 415 (1983), Seiten 159 - 175).

Technische Olefingemische, die als Edukte für die Oxo-Synthese verwendet werden, enthalten Olefinisomere der verschiedensten Strukturen mit unterschiedlichen Verzweigungsgraden, unterschiedlicher Lage der Doppelbindung im Molekül und gegebenenfalls auch unterschiedlichen Kohlenstoffzahlen. Dies gilt insbesondere für Olefingemische, die durch Di- oder Trimerisierung sowie weitergehende Oligomerisierung von C₂-C₅-Olefinen oder anderen leicht zugänglichen höheren Olefinen beziehungsweise durch Cooligomerisierung der genannten Olefine entstanden sind. Als Beispiele für typische isomere Olefingemische, die durch Rhodium-katalysierte oder vorzugsweise durch Kobalt-katalysierte Hydroformylierung zu den entsprechenden Aldehyd- und Alkohol-Gemischen umgesetzt werden können, seien Tri- und Tetrapropene sowie Di-, Tri- und Tetrabutene genannt.

Sind Alkohole mit möglichst geringem Verzweigungsgrad als Hydroformylierungsprodukt angestrebt, wird die Hydroformylierung vorteilhaft mit unmodifizierten Kobaltkatalysatoren durchgeführt. Im Vergleich zu Rhodiumkatalysatoren werden mit Kobaltkatalysatoren, ausgehend von einem gleichen Olefingemisch, höhere Ausbeuten an den besonders wertvollen geradkettigen Oxo-Produkten erhalten.

Die Hydroformylierung von Olefinen mit unmodifizierten Kobaltkatalysatoren kann, abgesehen von der Katalysatoraufarbeitung, ein- oder mehrstufig durchgeführt werden.

Die bekannten mehrstufigen Herstellungsverfahren von Oxo-Aldehyden in Gegenwart von unmodifizierten Kobaltkatalysatoren weisen eine Reihe von technischen Nachteilen auf. So sind für die Herstellung des für die Hydroformylierung benötigten Kobaltkatalysators zwei technisch aufwendige Verfahrensstufen, nämlich Vorcarbonylierung und Katalysatorextraktion, erforderlich. Bedingt durch die in den beiden Verfahrensstufen ablaufenden Stoffübergangsvorgänge, nämlich Gas/Flüssigkeit-Stoffübergang bei der Vorcarbonylierung und Flüssigkeit/Flüssigkeit-Stoffübergang bei der Katalysatorextraktion, sind zwei voneinander getrennte druckfeste Apparate, wie zum Beispiel Rührkessel oder Füllkörperkolonnen, erforderlich. Die eigentliche Hydroformylierung findet anschließend wiederum in einem separaten Druckreaktor statt.

Die deutsche Patentanmeldung DE 196 54 340 beschreibt ein Verfahren, in dem Vorcarbonylierung, Katalysatorextraktion und Olefin-Hydroformylierung in einem Reaktor durchgeführt werden. Dieser Prozess hat gegenüber den bekannten mehrstufigen Verfahren die Vorteile einer geringeren Kapitalanlage und geringerer Betriebskosten. Nachteilig ist jedoch, dass die Prozessführung des einstufigen Prozesses ziemlich schwierig ist, da die Verfahrensteilschritte wie Katalysatorbildung, Extraktion des Katalysators in die organische Phase und Hydroformylierung simultan erfolgen. Im Reaktor liegt im unteren Teil eine wässrige Kobaltsalzlösung vor, in der der Katalysator gebildet wird. Die Hydroformylierung geschieht hauptsächlich in der homogenen organischen Phase. Mit dem den Reaktor verlassenden Hydroformylierungsgemisch und dem abgezogenen Synthesegas werden kontinuierlich Wasser und Kobaltverbindungen aus dem Reaktor ausgetragen, so dass Kobaltverbindungen und Wasser ständig nachdosiert werden müssen.

Aus dem Hydroformylierungsprodukt dieses Prozesses werden darin enthaltener Kobalttetracarbonylwasserstoff und Dikobalttetracarbonylwasserstoff und sonstige vorhandenen Kobaltverbindungen mit einer Oxydationzahl von unter 2 mit Sauerstoff in Gegenwart einer wässrigen Kobalt(II)salzlösung zu Kobalt(II)salzen oxidiert. Die wässrige Kobaltsalzlösung wird von der organischen Phase getrennt, und eine Teilmenge davon als Katalysatorvorstufe in den Hydroformylierungsreaktor gefahren. Die wässrige Phase enthält hauptsächlich Kobaltformiat, weil Ameisensäure im Prozess entsteht, entweder direkt aus Kohlenmonoxid und Wasser oder durch Hydrolyse von als Nebenproduke entstandenen Alkylformiaten. Der Gehalt an Kobaltverbindungen in der (ameisen)sauren wässrigen Phase (pH =1 - 4) ist somit weitgehend von der Löslichkeit von Kobaltformiat vorgegeben. Der Gehalt an Kobaltverbindungen, berechnet als elementares Kobalt, kann bis etwa 1,7 Massen-% betragen.

Weiterhin ist die Menge an Kobalt, die in den Reaktor gebracht wird, von der Masse der Kobaltsalzlösung abhängig. Von dieser kann bei Verfahren, bei denen Katalysatorbildung, Katalysatorextraktion und Hydroformylierung gleichzeitig im gleichen Reaktor durchgeführt wird, wie etwa in DE 196 54 340 beschrieben, bei Konstanthalten der wässrigen Sumpfphase nur eine bestimmte Menge eingespeist werden. Die Menge an eingespeister wässriger Kobaltsalzlösung ist gerade so groß, dass die Menge des darin enthaltenen Wassers gerade der Menge des mit dem flüssigen Reaktionsgemisch und mit dem Gas aus dem Reaktor ausgetragenen Wassers entspricht.

Durch die beiden oben genannten Randbedingungen ergeben sich folgende Nachteile:

Bei der Hydroformylierung von Olefinen oder Olefingemischen mit mittleren oder hohen C-Zahlen (C-Zahl über acht) wird unter üblichen Hydroformylierungsbedingungen (Temperaturen über 160 °C und Betriebsdrücken über 200 bar), bezogen auf die eingespeiste Kobaltsalzlösung, bis zur Einstellung eines quasistationären Gleichgewichts anteilig mehr Kobalt(II)-salz zum aktiven Katalysator umgesetzt als Wasser mit der flüssigen organischen Phase und dem Gas aus dem Reaktor transportiert wird. Dies bedingt, dass die Kobaltkonzentration in der wässrigen Phase des Reaktors geringer als in der eingespeisten Lösung wird. Bei Temperaturen, bei denen die Geschwindigkeit für die Bildung des Katalysators relativ gering ist, ist dieser Konzentrationseffekt tolerierbar. Wird durch Anhebung der Temperatur die Geschwindigkeitskonstante für die Bildung des Katalysators erhöht, um mehr aktiven Katalysator zu erhalten, hemmt der Abfall der Konzentration an Kobaltsalzen in der wässrigen Phase dessen Bildung.

Bei der Hydroformylierung von höheren Olefinen oder Olefingemischen, die selbst bzw. deren durch Hydroformylierung entstandenen Produkte wenig oder kaum Wasser lösen, kann wegen der Wasserbilanz nur eine geringe Menge an Kobaltsalzen in den Reaktor gebracht werden. Dementsprechend gering ist die Menge an aktivem Katalysator und somit auch der Olefinumsatz. Im Grenzfall, wenn kein Wasser mit der organischen Phase und Gas aus dem Reaktor ausgetragen wird, ist ein kontinuierliches Verfahren, etwa gemäß DE 196 54 340, nicht durchführbar.

Es bestand daher die Aufgabe, den Einstufenprozess der Hydroformylierung, bestehend aus Katalysatorbildung, Katalysatorextraktion und Hydroformylierung im gleichen Reaktor, derart zu verbessern, dass die genannten Beeinträchtigungen des kontinuierlichen Betriebes vermieden und die Zielprodukte in hoher Ausbeute und in hoher Selektivität erhalten werden.

Es wurde nun gefunden, dass der Umsatz von Olefinen zu Hydroformylierungsprodukten bei der Hydroformylierung nach dem Einstufenprozess erhöht werden kann, wenn mehr wässrige Kobaltsalzlösung in den Reaktor eingespeist wird, als wässrige Phase mit dem flüssigen Reaktionsgemisch und dem Gas aus dem Reaktor ausgetragen wird, und zur Konstanthaltung des Stands der wässrigen Sumpfphase kontinuierlich ein Teil der Sumpfphase aus dem Reaktor gefahren wird.

Gegenstand der vorliegenden Erfindung ist ein einstufiges kontinuierliches Verfahren zur Hydroformylierung von Olefinen mit mindestens 5 Kohlenstoffatomen zu den entsprechenden Aldehyden und/oder Alkoholen mit mindestens 6 Kohlenstoffatomen in Gegenwart von unmodifizierten Kobaltkatalysatoren, bei dem bei Vorliegen einer wässrigen und einer organischen Phase Katalysatorbildung, Katalysatorextraktion und Hydroformylierung im gleichen Reaktor erfolgt, das dadurch gekennzeichnet ist, dass mehr wässrige Kobaltsalzlösung in den Reaktor eingespeist wird, als wässrige Phase mit dem flüssigen Reaktionsgemisch und mit der Gasphase aus dem Reaktor ausgetragen wird und dass zur Konstanthaltung des Stands der wässrigen Sumpfphase kontinuierlich ein Teil der wässrigen Sumpfphase aus dem Reaktor gefahren wird.

Das erfindungsgemäße Verfahren weist im Vergleich zu den bekannten Einstufenprozessen, beispielsweise nach DE 196 54 340, folgende Vorteile auf: Es werden bei gleicher Temperatur höhere Umsätze von Olefinen zu Hydroformylierungsprodukten erreicht oder bei gleichem Umsatz kann die Reaktionstemperatur gesenkt werden, wodurch die Selektivität für die Bildung von Hydroformylierungsprodukte erhöht wird. Weiterhin können auch Olefine nach dem Einstufenprozess hydroformyliert werden, die selbst und deren Hydroformylierungsprodukte nur eine geringe Löslichkeit für Wasser aufweisen.

Die vorliegende Erfindung ist ein einstufiges kontinuierliches Hydroformylierungsverfahren, bei dem die Bildung des aktiven Kobaltkatalysators aus einer wässrigen Kobaltsalzlösung, Extraktion des aktiven Katalysators aus der wässrigen Phase in die organische Phase und Hydroformylierung gleichzeitig im Reaktor abläuft, wobei mehr wässrige Kobaltsalzlösung in den Reaktor eingespeist wird, als wässrige Phase mit dem flüssigen Reaktionsgemisch und mit dem überschüssigen Synthesegas zusammen aus dem Reaktor ausgetragen wird, und der Überschuss an wässriger Phase durch kontinuierliches Abziehen eines Teil der wässrigen Unterphase entfernt wird.

Im erfindungsgemäßen Verfahren werden 2 bis 99 Massen-%, insbesondere 5 bis 80 Massen-% und ganz besonders 10 bis 60 Massen-% des in den Reaktor mit der Kobaltsalzlösung eingespeisten Wassers durch Abziehen eines Teils der wässrigen Unterphase entfernt. Der Anteil an Wasser, der mit der wässrigen Unterphase aus dem Reaktor abgezogen wird, ist hauptsächlich von den zu hydroformylierenden Olefinen und der Hydroformylierungstemperatur abhängig.

In einer homologen Reihe von Aldehyden oder Alkoholen, die bei einer Hydroformylierung entstehen können, nimmt das Lösevermögen für Wasser mit zunehmender Molmasse ab. Das hat zur Folge, dass mit steigender Molmasse der Einsatzolefine immer weniger Wasser mit dem flüssigen Reaktionsgemisch aus dem Reaktor transportiert wird, sodass ein zunehmender Anteil an Wasser durch Abziehen eines Teils der wässrigen Unterphase aus dem Reaktor entfernt werden muss. Die jeweils optimalen Bedingungen können von einem Fachmann durch Vorversuche leicht ermittelt werden.

Die Bildung des aktiven Kobaltkatalysators erfolgt bei Temperaturen unterhalb von 160 °C recht langsam. Bei Temperaturen oberhalb von 160 °C, insbesondere oberhalb von 180 °C dagegen bildet sich der Katalysator schnell, sodass die wässrige Lösung an Kobaltsalzen verarmt, wodurch die Bildung des aktiven Katalysators limitiert wird. Durch teilweisen Austausch der wässrigen Unterphase, die an Kobaltverbindungen verarmt ist, durch frische Kobaltsalzlösung kann die Katalysatorbildung erhöht werden.

Es ist also zweckmäßig, abhängig vom Einsatzolefin und der

Hydroformylierungstemperatur unterschiedliche Anteile des mit der wässrigen Kobaltsalzlösung in den Reaktor eingespeisten Wassers durch Abziehen eines Teils der wässrigen Unterphase zu entfernen.

Beispielsweise werden bei der Hydroformylierung von einem C₈-Olefingemisch, das im Wesentlichen aus n-Octenen, 3-Methylheptenen und 3,4-Dimethylhexenen besteht, im Temperaturbereich von 180 bis 190 °C 5 bis 70 % des in den Reaktor eingespeisten Wassers durch Abfahren eines Teils der Sumpfphase entfernt.

Die Hydroformylierung wird in einem Hochdruckreaktor, bevorzugt in einem kaskadierten Blasensäulenreaktor, durchgeführt, in den Olefine, eine wässrige Kobaltsalzlösung und Synthesegas, bevorzugt mittels einer Mischdüse eingebracht wird.

Der Stand der wässrigen Sumpfphase im Hydroformylierungsreaktor wird konstant oder nahezu konstant gehalten. Dies bedeutet, dass während eines stationären Betriebs bei konstanten Reaktionsbedingungen die Phasengrenze zwischen unterer wässriger Phase, in der ein Teil der organischen Phase dispergiert ist, und darüberliegender organischer Phase einen Stand annimmt, dessen Höhe bevorzugt weniger als ± 5 % um einen Mittelwert schwankt. Dieser Mittelwert der Höhe der Phasengrenze kann unterhalb oder oberhalb oder in Höhe der Austrittsöffnung der Mischdüse liegen, durch die die Edukte in den Reaktor gebracht werden. Die Phasengrenze kann sich dabei 0 bis 1 m, vorzugsweise 0 bis 0,5 m, besonders bevorzugt 0 bis 0,2 m über oder unter der Austrittsöffnung der Mischdüse befinden.

Die Höhe der wässrigen Phase kann sich während einer Laständerung innerhalb der Grenzen des oben genannten Bereichs verschieben. Weiterhin kann sich die Höhe der Wasserphase abhängig vom Durchsatz innerhalb dieser Grenzen verschieben.

Die wässrige Phase im unteren Teil des Reaktors beträgt 0,5 bis 20 %, insbesondere 1 bis 10 % des flüssigen Reaktorinhalts.

Erfindungsgemäß wird in dem Verfahren ein Teil der wässrigen Sumpfphase aus dem Reaktor gefahren. Dies kann schubweise oder bevorzugt kontinuierlich erfolgen. Vorzugsweise geschieht dies an einer Stelle, an der die Sumpfphase möglichst wenig bewegt wird und somit wenig dispergierte organische Phase enthält. Beispielsweise kann in einem Blasensäulenreaktor mit gewölbten Boden wässrige Phase aus dem Raum der Bodenwölbung unterhalb der Dosierdüse abgezogen werden.

In dem erfindungsgemäßen Verfahren wird in den Hydroformylierungsreaktor eine wässrige Lösung von Kobaltsalzen eingespeist. Bevorzugt werden wässrige Lösungen von Kobaltsalzen von Carbonsäuren, wie beispielsweise Kobaltformiat oder Kobaltacetat, eingesetzt. Es können auch Lösungen, die mehr als eine Kobaltverbindung enthalten, eingesetzt werden. Vorzugsweise ist die Kobaltlösung eine solche, die bei einer besonders bevorzugten Ausführung des Gesamtprozesses, nämlich bei der oxidativen Entkobaltung des Hydroformylierungsautrags, anfällt. Diese Lösung, die auch Ameisensäure enthält, kann direkt oder nach Aufkonzentrierung oder nach Reduzierung des Ameisensäuregehalts, beispielsweise wie in DE 100 09 207 beschrieben, eingesetzt werden.

Im erfindungsgemäßen Verfahren werden bevorzugt Lösungen eingesetzt, deren Kobaltsalzkonzentration größer als 30 %, insbesondere größer als 60 %, ganz besonders größer als 80 % der Sättigungskonzentration des Kobaltsalzes ist. Ist in der wässrigen Lösung hauptsächlich Kobaltformiat vorhanden, liegt der Gehalt an Kobaltsalzen, berechnet als elementares Kobalt, vorzugsweise im Bereich von 0,7 bis 1,7 Massen-%.

Die Hydroformylierung der vorliegenden Erfindung wird ähnlich betrieben, wie in DE 196 54 340 und DE 101 35 906 dargelegt. Der Unterschied liegt darin, dass erfindungsgemäß mehr wässrige Kobaltsalzlösung zudosiert und ein Teil der wässrigen Unterphase entnommen wird.

Die Hydroformylierung wird im Temperaturbereich von 110 bis 250 °C, bevorzugt im Temperaturbereich von 130 bis 220 °C, ganz besonders bevorzugt im Temperaturbereich von 160 °C bis 190 °C durchgeführt.

Der Reaktionsdruck liegt im Bereich von 100 bis 400 bar, insbesondere im Bereich von 150 bis 300 bar. Im eingesetzten Synthesegas, dem Gemisch aus Wasserstoff und Kohlenstoffmonoxid, liegt das Volumenverhältnis von Wasserstoff zu Kohlenstoffmonoxid vorzugsweise im Bereich 1 zu 2 bis 2 zu 1.

In der vorliegenden Erfindung werden das Einsatzolefin oder Olefingemisch, die wässrige Lösung mit den Kobaltverbindungen und Synthesegas und gegebenenfalls ein Lösemittel in den Sumpf des Hydroformylierungsreaktor eingebracht. Im Sumpf des Reaktors liegt eine wässrige Phase vor, in der geringe Mengen an organischer Phase dispergiert sind.

Um eine hohe Reaktionsgeschwindigkeit zu erhalten, ist es zweckmäßig, die wässrige Sumpfphase mit der organischen Phase und Synthesegas sowie der wässrigen Kobaltsalzlösung zu vermischen. Durch die intensive Vermischung werden Konzentrationsgradienten der Reaktionspartner vermieden. Darüber hinaus begünstigt die Vermischung der wässrigen Sumpfphase mit der organischen Phase den Übergang des gebildeten Katalysators in die organische Phase, in der die Hydroformylierung hauptsächlich abläuft.

Die Vermischung der Reaktionskomponenten Olefin, Synthesegas, wässrige Kobaltsalzlösung mit sich selbst und/oder Hydroformylierungsgemisch sowie die Vermischung der beiden flüssigen Phasen im Reaktor kann mit Hilfe geeigneter technischer Einrichtungen erfolgen.

Olefin, Synthesegas und wässrige Kobaltsalzlösung können getrennt, zweckmäßig durch Düsen, in den Reaktor eingeleitet werden. Es können auch zwei Komponenten zusammen durch eine oder mehrere Mischdüsen und die dritte Komponente getrennt in den Reaktor eingespeist werden. Zweckmäßig ist es jedoch, alle drei Komponenten zusammen durch eine oder mehrere Mischdüsen dem Reaktor zuzuführen.

Die wässrige Sumpfphase kann mit Hilfe einer Pumpe, die in einer Umlaufleitung installiert ist, umgewälzt werden. Eine Durchmischung der wässrigen Phase und Vermischung der wässrigen Phase mit der organischen Phase und Synthesegas kann auch dadurch erreicht werden, dass ein Teil der wässrigen Phase aus dem Reaktor der Mischdüse für die Einsatzstoffe zugeleitet wird. Dies kann mit Hilfe einer Pumpe geschehen. Eine andere Möglichkeit besteht darin, einen Teil der wässrigen Sumpfphase aus dem Reaktor von der Stoffströmung in der Mischdüse ansaugen zu lassen.

Die Ejektorwirkung von Mischdüsen wird durch den Impuls des austretenden Gases und der austretenden Flüssigkeit beeinflusst. Hohe Flüssigkeitsgeschwindigkeiten von 3 bis 300 m/s, besonders 10 bis 100 m/s, ganz besonders von 15 bis 70 m/s am Ort beziehungsweise an den Orten der Einmischung werden bevorzugt.

Das Reaktionsgemisch aus dem Hydroformylierungsreaktor enthält Einsatzstoff (Olefine), Produkte (Aldehyde, Alkohole, Ameisensäureester), Nebenprodukte und Kobaltcarbonylverbindungen. Die letzteren können aus dem Reaktionsgemisch nach an sich bekannten technischen Verfahren abgetrennt werden. Bevorzugt erfolgt die Abtrennung der Kobaltcarbonyle oxidativ. Dazu wird das Reaktionsgemisch teilentspannt, insbesondere auf 10 bis 15 bar, und in Gegenwart von einer sauren Kobalt(II)salzlösung mit Sauerstoff enthaltenen Gasen, insbesondere Luft oder Sauerstoff, bei Temperaturen von 90 °C bis 160 °C in einem Reaktor (Entkobalter) umgesetzt und so oxidativ von Kobalt-Carbonylverbindungen befreit. Diese werden dabei unter Bildung von Kobalt(II)salzen zerstört. Die Entkobaltungsverfahren sind gut bekannt und in der Literatur ausführlich, wie z. B. in "New Syntheses with Carbon Monoxide", Springer Verlag (1980), Berlin, Heidelberg, New York, Seite 158 f., beschrieben. Nach der Oxidation wird das Gemisch in die organische Produktphase, Abgas und Prozesswasser getrennt. Das abgetrennte Prozesswasser weist typisch einen pH-Wert von 1,5 bis 4,5 und einen Kobaltgehalt zwischen 0,5 und 2 Massen-% auf. Der größte Teil des Prozesswassers wird gegebenenfalls unter Zusatz von Wasser in den Entkobalter zurückgefahren. Der andere Teil wird vorzugsweise in den Hydroformylierungsreaktor zurückgefahren.

Die aus dem Hydroformylierungsreaktor abgezogene wässrige Sumpfphase enthält neben Kobalt(II)salzen, organische Substanzen und Kobaltcarbonylverbindungen. Wenn diese Lösung als solche nicht verwendet werden kann, ist es zweckmäßig, diese zusammen mit dem Hydroformylierungsgemisch in den Entkobalter zu leiten. Vorzugsweise kann der wässrige Sumpfabzug, bevor er weiterverwendet oder aufgearbeitet wird, mit Einsatzolefin extrahiert werden, wobei ein Teil der Kobaltcarbonyle und der organischen Substanzen darin von der Olefinphase aufgenommen wird. Die mit Kobaltcarbonylen beladene Olefinphase wird in den Hydroformylierungsreaktor gefahren. Die Extraktion wird vorzugsweise im Gegenstrom in üblichen technischen Extraktionsapparaturen, beispielsweise in einer mit Füllkörpern gefüllten Kolonne, durchgeführt. Die Extraktionstemperatur kann zwischen 20 und 220 °C liegen. Der Druck in der Reaktionskolonne kann 1 bis 400 bar betragen.

Das nach der Abtrennung der Kobaltcarbonyle enthaltene organische Reaktionsgemisch wird nach bekannten Verfahren aufgearbeitet. Beispielsweise kann es durch Destillation in eine Kohlenwasserstofffraktion, die nicht umgesetzte Olefine enthalten kann, in Aldehyde, andere Wertprodukte und sonstige Stoffe getrennt werden. Die Kohlenwasserstofffraktion mit nicht umgesetzten Olefinen kann teilweise in die gleiche erfindungsgemäße Hydroformylierung zurückgeführt werden oder in einer weiteren Hydroformylierung, die auch erfindungsgemäß durchgeführt sein kann, umgesetzt werden. Die gewonnen Aldehyde können als solche verwendet werden oder können Ausgangsstoff für die Herstellung anderer Stoffe sein, beispielsweise Carbonsäuren, Amine, Nitrile oder Aldolkondensationsprodukte. Weiterhin kann das Hydroformylierungsgemisch vor oder nach Abtrennung der nicht umgesetzten Olefine zu den entsprechenden primären Alkoholen hydriert werden, die u. a. Vorstufen für Weichmacher oder Detergenzien sein können.

Als Edukte für das erfindungsgemäße Verfahren können in Prinzip alle Olefine mit mindestens 5 Kohlenstoffatomen eingesetzt werden. Die eingesetzten Edukte können lineare oder verzweigte α-Olefine, lineare oder verzweigte Olefine mit innenständigen Doppelbindungen, cycloaliphatische Olefine oder Olefine mit aromatischen Gruppen sein. Es können Stoffe mit einer oder mehreren olefinischen Doppelbindung(en) eingesetzt werden. Vorzugsweise werden Olefine oder Olefingemische mit 6 bis 24 Kohlenstoffatomen eingesetzt. Die Gemische können aus Olefinen gleicher, ähnlicher (+ 2) oder deutlich unterschiedlicher (> + 2) C-Zahl bestehen. Als Olefine, die entweder in reiner Form, in einem Isomerengemisch oder in einem Gemisch mit weiteren Olefinen anderer C-Zahl als Edukt eingesetzt werden können, seien beispielsweise genannt: 1-, 2- oder 3-Hexen, 1-Hepten, lineare Heptene mit innenständiger Doppelbindung (2-Hepten, 3-Hepten usw.), Gemische linearer Heptene, 2- oder 3-Methyl-1-hexen, 1-Octen, lineare Octene mit innenständiger Doppelbindung, Gemische linearer Octene, 2- oder 3-Methylhepten, 1-Nonen, lineare Nonene mit innenständiger Doppelbindung, Gemische linearer Nonene, 2-, 3- oder 4-Methyloctene, 1-, 2-, 3-, 4- oder 5-Decen, 2-Ethyl-1-octen, 1-Dodecen, lineare Dodecene mit innenständiger Doppelbindung, Gemische linearer Dodecene, 1-Tetradecen, lineare Tetradecene mit innenständiger Doppelbindung, Gemische linearer Tetradecene, 1-Hexadecen, lineare Hexadecene mit innenständiger Doppelbindung, Gemische linearer Hexadecene. Geeignete Edukte sind weiterhin u. a. das bei der Dimerisierung von Propen anfallende Gemisch isomerer Hexene (Dipropen), das bei der Dimerisierung von Butenen anfallende Gemisch isomerer Octene (Dibuten), das bei der Trimerisierung von Propen anfallende Gemisch isomerer Nonene (Tripropen), das bei der Tetramerisierung von Propen oder der Trimerisierung von Butenen anfallende Gemisch isomerer Dodecene (Tetrapropen oder Tributen), das bei der Tetramerisierung von Butenen anfallende Hexadecen-Gemisch (Tetrabuten) sowie durch Cooligomerisierung von Olefinen mit unterschiedlicher C-Zahl (bevorzugt 2 bis 4) hergestellte Olefingemische, gegebenenfalls nach destillativer Trennung in Fraktionen mit gleicher oder ähnlicher (±2) C-Zahl. Weiterhin können Olefine oder Olefingemische, die durch Fischer-Tropsch-Synthese erzeugt worden sind, eingesetzt werden. Darüber hinaus können Olefine, die durch Olefin-Metathese oder durch andere technische Prozesse hergestellt worden sind, verwendet werden. Bevorzugte Edukte sind Gemische isomerer Octene-, Nonene-, Dodecene- oder Hexadecene, d. h. Oligomere von niedrigen Olefinen, wie n-Butenen, Isobuten oder Propen. Andere ebenfalls gut geeignete Edukte sind Oligomere aus C₅-Olefinen. Darüber hinaus ist das erfindungsgemäße Verfahren auch für die Hydroformylierung von polymeren Olefinen, beispielsweise Polyisobuten, Polybutadien, 1,3-Butadien-Isobuten- oder Buten-Copolymere mit einer Molmasse bis zu 6000 Dalton, gegebenenfalls unter Verwendung eines Verdünnungsmittel, geeignet.

Wenn C₈-, C₁₂- oder C₁₆- Olefingemische die Edukte sind, werden insbesondere solche eingesetzt, die durch Oligomerisierung von linearen Butenen an Nickel-Festbettkatalysatoren hergestellt worden sind, wie beispielsweise nach dem Octol-Prozess (Hydrocarbon Process, Int. Ed. (1986) 65 (2.Sect. 1) Seite 31 - 33).

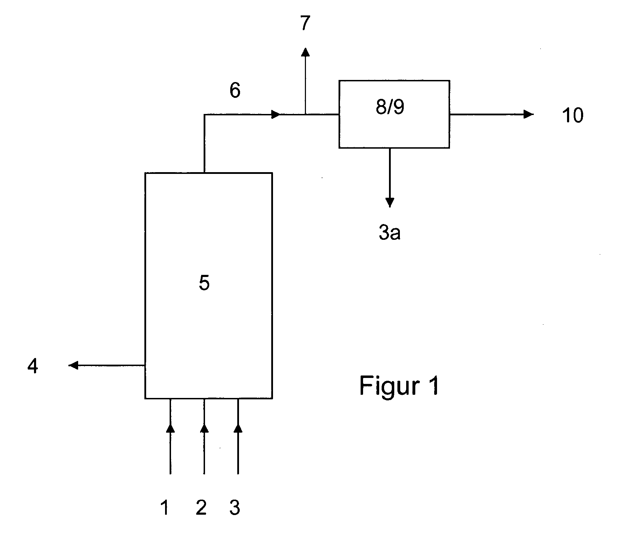

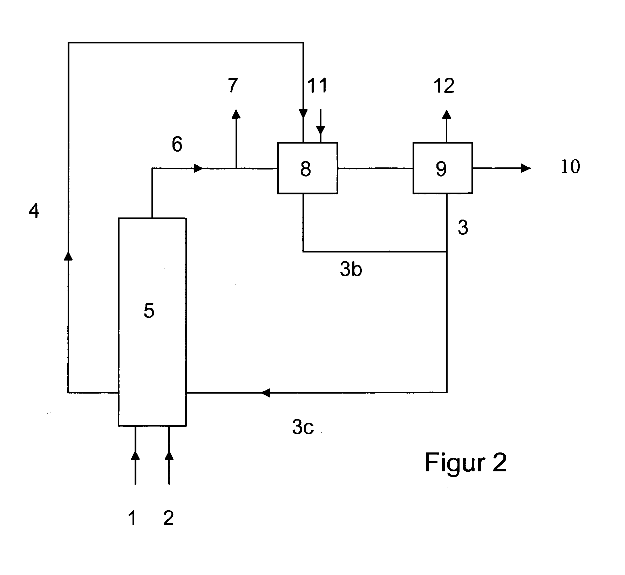

Anhand der Figuren 1 und 2 wird die Erfindung erläutert. An sich selbstverständliche Anlagedetails, die für das Verständnis der Erfindung nicht erforderlich sind, sind der Übersichtlichkeit halber weggelassen.

Figur 1 zeigt die schematische Darstellung der einfachsten Art einer Anlage zur Durchführung des erfindungsgemäßen Verfahrens. Diese Anlage weist keinen geschlossenen Katalysatorkreislauf auf. Einsatzolefin (1), Synthesegas (2) und wässrige Kobalt(II)salzlösung (3) werden in den Hydroformylierungsreaktor (5) gefahren, in dem eine wässrige Unterphase vorhanden ist. Ein Teil (4) der wässrigen Sumpfphase wird abgezogen. Das Hydroformylierungsgemisch (6) wird, gegebenenfalls nach Abziehen von überschüssigem Synthesegas (7) einer Entkobaltung (8/9) zugeführt. Es wird ein Strom (3a) mit Kobaltverbindungen abgetrennt und ein praktisch Kobalt-freies Hydroformylierungsgemisch (10) erhalten.

Figur 2 ist die schematische Darstellung einer Anlage, in der das erfindungsgemäße Verfahren in einer bevorzugten Ausführungsform durchgeführt werden kann. Olefin (1), Synthesegas (2) und eine wässrige Kobaltsalzlösung (3c) werden in den Hydroformylierungsreaktor (5) gefahren, in dem eine wässrige Unterphase vorhanden ist. Ein Teil (4) der wässrigen Sumpfphase wird abgezogen. Der Austrag (6) aus dem Hydroformylierungsreaktor (5) wird teilentspannt und dabei ein Teil (7) des überschüssigen Synthesegas abgetrennt. Im Oxidator (8) wird das teilenspannte Hydroformylierungsgemisch (6) zusammen mit dem Sumpfabzug (4) mit einem Sauerstoff enthaltenen Gas (11) in Gegenwart des Prozesswassers (3b) umgesetzt, wobei Kobaltcarbonyle zu Kobalt(II)salzen oxidiert werden. Im Trenngefäß (9) erfolgt die Trennung in Abgas (12), Hydroformylierungsgemisch (10) und Prozesswasser (3). Ein Teil (3b) des Prozesswassers wird in den Oxidator (8) und der andere Teil (3c) in den Hydroformylierungsreaktor (5) zurückgeführt. Das Hydroformylierungsgemisch (10) wird nach bekannten Verfahren aufgearbeitet. Die Wassermenge und die Kobaltspuren, die mit dem Strom (10) ausgetragen werden, werden ergänzt, beispielsweise durch Zudosierung der entsprechenden Mengen in den Behälter (9).

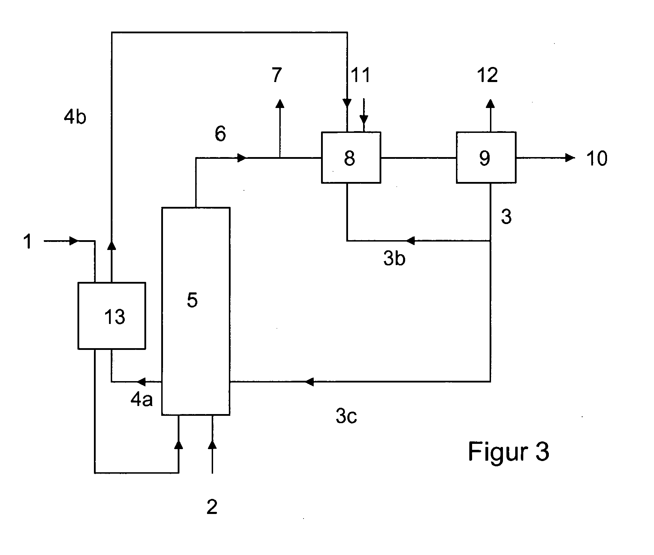

Figur 3 zeigt eine weitere Ausführung des erfindungsgemäßen Verfahrens. Diese Ausführung unterscheidet sich von der Variante gemäß Figur 2 dadurch, dass die aus dem Reaktor abgezogene wässrige Unterphase (4a) im Extraktor (13) mit Einsatzolefin (1) extraktiert wird.

Die folgenden Beispiele sollen die Erfindung erläutern, ohne diese darauf zu beschränken.

### Beispiel 1: Hydroformylierung von C₈-Olefinen ohne Ausschleusung von Kobalt-Wasser (nicht erfindungsgemäß)

Für die kontinuierliche Hydroformylierung des C₈-Olefingemisches wurde eine Versuchsanlage verwendet, die im Wesentlichen aus einem senkrecht stehendem Hochdruckrohrreaktor (90 mm innerer Durchmesser; Länge 3600 mm) und einem nachgeschalteten, mit Raschigringen befüllten Entkobaltungsbehälter (20 l Fassungsvermögen) sowie einen Phasentrennbehälter bestand. Durch fünf, orthogonal zur Strömungsrichtung eingebaute Lochbleche war der Reaktionsraum des Hochdruckreaktors kaskadiert. Für die Dosierung der Einsatzstoffe Olefingemisch, Synthesegas und wässrige Katalysatorlösung wurde eine Dreistoff-Mischdüse verwendet. Der Reaktorinhalt konnte nach Bedarf durch installierte Heiz- und Kühlvorrichtungen aufgeheizt oder gekühlt werden.

Die Bildung des aktiven Kobalt-Katalysators, die Extraktion des aktiven Kobalt-Katalysators in die Olefinphase und die Hydroformylierung liefen simultan im Hochdruckreaktor ab.

Als C₈-Olefingemisch wurde ein Di-n-Butengemisch, hergestellt nach dem Octol-Prozess der Oxeno GmbH, verwendet.

Der Reaktor wurde mit Dibuten gefüllt und auf die Reaktionstemperatur von 180 °C gebracht. Anschließend wurden dem Reaktorsumpf über die Mischdüse Dibuten, wässrige Kobaltacetatlösung mit 1 Massen-% Kobalt, berechnet als Element, und Synthesegas mit einem CO/H₂-Volumenverhältnis von 1 zu 1 kontinuierlich zugeführt.

Folgende Durchsätze wurden eingestellt: 5,0 kg/h Dibuten und 0,35 kg/h Kobaltacetat-Lösung. Der Reaktor wurde mit Synthesegas auf einen konstanten Reaktionsdruck von 280 bar bei einem Synthesegas-Durchsatz von 2,5 bis 3,5 Nm³/h druckgeregelt.

Das Hydroformylierungsgemisch wurde am Reaktorkopf kontinuierlich abgezogen und in der Entkobaltungsstufe auf 15 bar entspannt.

Im unteren Teil des Reaktors bildete sich eine wässrige Sumpfphase, deren Stand nach Erreichen des quasistationären Gleichgewichts unverändert blieb. Dazu wurde gerade so viel Wasser und Kobalt in den Reaktor eingeleitet, wie mit dem flüssigen Hydroformylierungsgemisch und überschüssigen Synthesegas aus dem Reaktor entfernt wurden.

In der Entkobaltungsstufe wurde das Hydroformylierungsgemisch in Gegenwart von saurer Kobaltsalzlösung durch Oxidation der Kobaltcarbonylkomplexe mit Luft vom aktiven Kobaltkatalysator befreit. Die beiden Phasen wurden anschließend im nachgeschalteten Trennbehälter getrennt.

Das entkobaltete Reaktionsprodukt wies nach 24 h Betriebsdauer nach GC-Analyse folgende Zusammensetzung in Massen-% auf: 5,1 C₈-Olefine, 3,1 C₈-Paraffine, 29,9 Isononanale, 53,8 Isononanole, 3,9 Ester (Nonylformiate) und 4,2 Hochsieder. Daraus berechneten sich ein Dibuten-Umsatz von 93,5 % und eine Wertproduktselektivität von 91,2 %. (Wertprodukte sind Isononanale, Isononanole und Nonylformiate.)

### Beispiel 2: Hydroformylierung von Dibuten unter Ausschleusung von Kobaltwasser (erfindungsgemäß)

Der Versuch gemäß Beispiel 1 wurde fortgeführt. Dabei wurde nur der Durchsatz an Kobaltacetat-Lösung von 0,35 kg/h auf 0,50 kg/h erhöht. Alle anderen Bedingungen wurden konstant gehalten. Um den Stand der wässrigen Phase ebenfalls konstant zu halten, mussten nach Erreichen des quasistationären Zustands 0,15 kg/h Prozesswasser aus dem Reaktor abgezogen werden, das in den Entkobalter geleitet wurde.

Das entkobaltete Reaktionsgemisch wies nach 24 h nach GC-Analyse folgende Zusammensetzung in Massen-% auf: 4,4 C₈-Olefine, 2,7 C₈-Paraffine, 30,7 Isononanale, 54,1 Isononanole, 4,0 Ester (Isononylformiate) und 4,1 Hochsieder. Daraus berechneten sich ein Dibuten-Umsatz von 94,5 % und eine Wertproduktselektivität von 92,0 %.

Es zeigt sich, dass durch die erfindungsgemäße Maßnahme sowohl der Dibuten-Umsatz als auch die Wertproduktselektivität erhöht werden.

## Patentansprüche

1. Einstufiges kontinuierliches Verfahren zur Hydroformylierung von Olefinen mit mindestens 5 Kohlenstoffatomen zu den entsprechenden Aldehyden und/oder Alkoholen mit mindestens 6 Kohlenstoffatomen in Gegenwart von unmodifizierten
Kobaltkatalysatoren, bei dem bei Vorliegen einer wässrigen und einer organischen Phase Katalysatorbildung, Katalysatorextraktion und
Hydroformylierung im gleichen Reaktor erfolgt,
**dadurch gekennzeichnet,**
**dass** mehr wässrige Kobaltsalzlösung in den Reaktor eingespeist wird, als wässrige Phase mit dem flüssigen Reaktionsgemisch und der Gasphase aus dem Reaktor ausgetragen wird und dass zur Konstanthaltung des Stands der wässrigen Sumpfphase kontinuierlich ein Teil der wässrigen Sumpfphase aus dem Reaktor gefahren wird.

2. Verfahren nach Anspruch 1,
**dadurch gekennzeichnet,**
**dass** 2 bis 99 Massen-% des in den Reaktor eingespeisten Wassers durch Abziehen eines Teils der wässrigen Unterphase entfernt wird.

3. Verfahren nach Anspruch 1,
**dadurch gekennzeichnet,**
**dass** 5 bis 80 Massen-% des in den Reaktor eingespeisten Wassers durch Abziehen eines Teils der wässrigen Unterphase entfernt wird.

4. Verfahren nach Anspruch 1,
**dadurch gekennzeichnet,**
**dass** 10 bis 60 Massen-% des in den Reaktor eingespeisten Wassers durch Abziehen eines Teils der wässrigen Unterphase entfernt wird.

5. Verfahren nach den Ansprüchen 1 bis 4,
**dadurch gekennzeichnet,**
**dass** der Stand der wässrigen Sumpfphase im Hydroformylierungsreaktor konstant oder nahezu konstant gehalten wird, wobei während des stationären Betriebs bei konstanten Reaktionsbedingungen die Phasengrenze zwischen unterer wässriger Phase und darüberliegender organischer Phase einen Stand annimmt, dessen Höhe weniger als ± 5 % um einen Mittelwert schwankt.

6. Verfahren nach Anspruch 5,
**dadurch gekennzeichnet,**
**dass** der Mittelwert der Höhe der Phasengrenze in Höhe der Austrittsöffnung der Mischdüse liegt, durch die die Edukte in den Reaktor gebracht werden.

7. Verfahren nach Anspruch 6,
**dadurch gekennzeichnet,**
**dass** die Phasengrenze sich 0 bis 1 m, vorzugsweise 0 bis 0,5 m, besonders bevorzugt 0 bis 0,2 m über oder unter der Austrittsöffnung der Mischdüse befindet.

8. Verfahren nach den Ansprüchen 1 bis 7,
**dadurch gekennzeichnet,**
**dass** die abgezogene Unterphase mit Einsatzolefin extrahiert wird.

9. Verfahren nach den Ansprüchen 1 bis 8,
**dadurch gekennzeichnet,**
**dass** die in den Reaktor eingespeiste wässrige Kobaltsalzlösung im Wesentlichen Kobaltformiate enthält.

10. Verfahren nach den Ansprüchen 1 bis 9,
**dadurch gekennzeichnet,**
**dass** die Konzentration der Kobaltsalze in der wässrigen Lösung, die in den Reaktor eingespeist wird, größer als 30 %, vorzugsweise größer als 60 %, besonders bevorzugt größer als 80 % der Sättigungskonzentration ist.

11. Verfahren den Ansprüchen 1 bis 10,
**dadurch gekennzeichnet,**
**dass** Olefingemische mit 6 bis 24 C-Atomen eingesetzt werden.

12. Verfahren nach Anspruch 11,
**dadurch gekennzeichnet,**
**dass** Olefingemische mit 8 oder mit 12 oder mit 16 C-Atomen eingesetzt werden.

13. Verfahren nach Anspruch 11,
**dadurch gekennzeichnet,**
**dass** Olefingemische, hergestellt durch Oligomerisierung von linearen Butenen an Nickel-Festbettkatalysatoren, eingesetzt werden.

## Claims

1. Single-stage continuous process for the hydroformylation of an olefin having at least 5 carbon atoms to the corresponding aldehyde and/or alcohol having at least 6 carbon atoms in the presence of unmodified cobalt catalysts, in which catalyst formation, catalyst extraction and hydroformylation occur in the same reactor in which an aqueous phase and an organic phase are present, **characterized in that** the amount of aqueous cobalt salt solution fed into the reactor is greater than the amount discharged from the reactor as aqueous phase with the liquid reaction mixture and the gas phase and part of the aqueous bottom phase is discharged continuously from the reactor to keep the level of the aqueous bottom phase constant.

2. Process according to Claim 1,
**characterized in that**
from 2 to 99% by mass of the water fed into the reactor is removed by taking off part of the aqueous bottom phase.

3. Process according to Claim 1,
**characterized in that**
from 5 to 80% by mass of the water fed into the reactor is removed by taking off part of the aqueous bottom phase.

4. Process according to Claim 1,
**characterized in that**
from 10 to 60% by mass of the water fed into the reactor is removed by taking off part of the aqueous bottom phase.

5. Process according to any of Claims 1 to 4, **characterized in that**
the level of the aqueous bottom phase in the hydroformylation reactor is kept constant or virtually constant, with the phase boundary between the lower aqueous phase and the organic phase above it during steady-state operation under constant reaction conditions being at a level whose height preferably fluctuates by less than ± 5% about a mean.

6. Process according to Claim 5,
**characterized in that**
the mean height of the phase boundary is at the height of the outlet opening of the mixing nozzle through which the starting materials are introduced into the reactor.

7. Process according to Claim 6,
**characterized in that**
the phase boundary is located from 0 to 1 m, preferably from 0 to 0.5 m, particularly preferably from 0 to 0.2 m, above or below the outlet opening of the mixing nozzle.

8. Process according to any of Claims 1 to 7, **characterized in that**
the bottom phase taken off is extracted with starting olefin.

9. Process according to any of Claims 1 to 8, **characterized in that**
the aqueous cobalt salt solution fed into the reactor contains essentially cobalt formates.

10. Process according to any of Claims 1 to 9, **characterized in that**
the concentration of cobalt salts in the aqueous solution fed into the reactor is greater than 30%, preferably greater than 60%, particularly preferably greater than 80%, of the saturation concentration.

11. Process according to any of Claims 1 to 10, **characterized in that**
an olefin mixture having from 6 to 24 carbon atoms is used.

12. Process according to Claim 11,
**characterized in that** an olefin mixture having 8 or 12 or 16 carbon atoms is used.

13. Process according to Claim 11,
**characterized in that** an olefin mixture prepared by oligomerization of linear butenes over fixed-bed nickel catalysts is used.

## Revendications

1. Procédé continu à une étape d'hydroformylation d'oléfines contenant au moins 5 atomes de carbone en les aldéhydes et/ou alcools correspondants contenant au moins 6 atomes de carbone en présence de catalyseurs de cobalt non modifiés, selon lequel la formation du catalyseur, l'extraction du catalyseur et l'hydroformylation ont lieu dans le même réacteur en présence d'une phase aqueuse et d'une phase organique, **caractérisé en ce que**
davantage de solution aqueuse de sel de cobalt est introduite dans le réacteur que de phase aqueuse est déchargée du réacteur avec le mélange réactionnel liquide et la phase gazeuse, et **en ce qu'**une partie de la phase aqueuse de fond est déchargée du réacteur en continu pour maintenir constant le niveau de la phase aqueuse de fond.

2. Procédé selon la revendication 1, **caractérisé en ce que** 2 à 99 % en masse de l'eau introduite dans le réacteur est éliminée par soutirage d'une partie de la phase aqueuse inférieure.

3. Procédé selon la revendication 1, **caractérisé en ce que** 5 à 80 % en masse de l'eau introduite dans le réacteur est éliminée par soutirage d'une partie de la phase aqueuse inférieure.

4. Procédé selon la revendication 1, **caractérisé en ce que** 10 à 60 % en masse de l'eau introduite dans le réacteur est éliminée par soutirage d'une partie de la phase aqueuse inférieure.

5. Procédé selon les revendications 1 à 4, **caractérisé en ce que** le niveau de la phase aqueuse de fond dans le réacteur d'hydroformylation est maintenu constant ou presque constant, l'interface entre la phase aqueuse inférieure et la phase organique susjacente ayant un niveau dont la hauteur oscille de moins de ± 5 % autour d'une valeur moyenne pendant l'exploitation stationnaire dans des conditions de réaction constantes.

6. Procédé selon la revendication 5, **caractérisé en ce que** la valeur moyenne de la hauteur de l'interface se situe à la hauteur de l'ouverture de sortie de la buse de mélange par laquelle les réactifs sont introduits dans le réacteur.

7. Procédé selon la revendication 6, **caractérisé en ce que** l'interface se trouve 0 à 1 m, de préférence 0 à 0,5 m, de manière particulièrement préférée 0 à 0,2 m au-dessus ou en dessous de l'ouverture de sortie de la buse de mélange.

8. Procédé selon les revendications 1 à 7, **caractérisé en ce que** la phase inférieure soutirée est extraite avec une oléfine d'alimentation.

9. Procédé selon les revendications 1 à 8, **caractérisé en ce que** la solution aqueuse de sel de cobalt introduite dans le réacteur contient essentiellement des formiates de cobalt.

10. Procédé selon les revendications 1 à 9, **caractérisé en ce que** la concentration des sels de cobalt dans la solution aqueuse introduite dans le réacteur est supérieure à 30 %, de préférence supérieure à 60 %, de manière particulièrement préférée supérieure à 80 %, de la concentration de saturation.

11. Procédé selon les revendications 1 à 10, **caractérisé en ce que** des mélanges d'oléfines contenant 6 à 24 atomes C sont utilisés.

12. Procédé selon la revendication 11, **caractérisé en ce que** des mélanges d'oléfines contenant 8 ou 12 ou 16 atomes C sont utilisés.

13. Procédé selon la revendication 11, **caractérisé en ce que** des mélanges d'oléfines fabriqués par oligomérisation de butènes linéaires sur des catalyseurs en lit fixe de nickel sont utilisés.
